# EUROPEAN PATENT APPLICATION

(11) **EP 2 495 547 A1**
(43) Date of publication of application: **05.09.2012**
(21) Application number: 12157815.7
(22) Date of filing: 01.03.2012
(51) Int. Cl.: G01N 21/35, G01N 33/28

(54) **Detecting ethanol and water concentration in fuel**

(30) Priority: 03.03.2011 US 201113039960
(71) Applicant: Honeywell International, Inc., Morristown, NJ 07962-2245 (US)
(72) Inventor: Aavramescu, Viorel, Morristown, NJ New Jersey 07962-2245 (US); Le Moing, Christophe, Morristown, NJ New Jersey 07962-2245 (US); Gierczak, Marek, Morristown, NJ New Jersey 07962-2245 (US); Schweizer, Pascal, Morristown, NJ New Jersey 07962-2245 (US); Buiu, Octavian, Morristown, NJ New Jersey 07962-2245 (US)
(74) Representative: Buckley, Guy Julian

(57) **Abstract**

A device (10) comprises a fuel line (5) that carries a combustible fuel including gasoline, a first optical channel (2,4) that evaluates a degree of absorption at a first wavelength spectrum of light transmitted through the combustible fuel within the fuel line, and a second optical channel (3,4) that evaluates a degree of absorption at a second wavelength spectrum. The first and second wavelength spectrums consists of wavelengths of between about 800 nanometers (nm) and about 1200 nm. The device further comprises a controller (7) configured to receive inputs from the first and second optical channels representing the degrees of absorption at the first and second wavelength spectrums, correlate the degrees of absorption with proportions of ethanol and water in the combustible fuel, and output data corresponding to the proportions of ethanol and water to a controller of a combustion engine fed with the combustible fuel.

## Description

### TECHNICAL FIELD

This disclosure relates to detecting compositions in hydrocarbon-based fuels.

### BACKGROUND

An internal combustions (IC) engine, such as an IC engine for an automobile or other vehicle may operate on different blends of fuel during its lifetime. Different fuels may include different concentrations of ethanol or other additives or ingredients. Some IC engines are designed to operate under a wide range of ethanol concentrations in gasoline-ethanol blends, such as from 0-85 percent ethanol. Automobiles designed to operate with high-level gasoline-ethanol blends of between 60-85 percent ethanol are commonly referred to as flexible-fuel vehicles (FFVs). In many areas, low-level gasoline-ethanol blends with ethanol concentrations of up to 10 percent are either available or even legally mandated for use in automobiles. For this reason, substantially all IC engines for automobiles should be able to operate with gasoline-ethanol blends of between 0-10 ethanol. Different gasoline-ethanol blends provide different energy concentrations, emissions and octane ratings (a measure of a fuel's resistance to pre-ignition or engine "knock").

### SUMMARY

In general, this disclosure relates to detecting concentrations of ethanol and water in gasoline-ethanol blends. In one example, concentrations of ethanol and water in gasoline-ethanol blends used to operate an IC engine may be monitored during the operation of the IC engine and used to actively adjust operational parameters of the IC engine. Actively adjusting operational parameters of the IC engine and/or associated emissions systems based on monitored concentrations of ethanol and/or water may facilitate reducing the emissions level and improvement in the overall performance of the IC, such as, efficiency, and/or reliability.

In one example, a device comprises a fuel line that carries a combustible fuel including gasoline, a first optical channel that evaluates a degree of absorption at a first wavelength spectrum of light transmitted through the combustible fuel within the fuel line, and a second optical channel that evaluates a degree of absorption at a second wavelength spectrum of light transmitted through the combustible fuel within the fuel line. The first and second wavelength spectrums each consists of wavelengths of between about 800 nanometers (nm) and about 1200 nm. The device further comprises a controller configured to receive inputs from the first and second optical channels representing the degree of absorption at the first wavelength spectrum and the degree of absorption at the second wavelength spectrum, correlate the degree of absorption at the first wavelength spectrum and the degree of absorption at the second wavelength spectrum with a proportion of ethanol in the combustible fuel and a proportion of water in the combustible fuel, and output data corresponding to the proportions of ethanol and water in the combustible fuel to a controller.

In another example, a method comprises evaluating a degree of absorption at a first wavelength spectrum of light transmitted through a combustible fuel including gasoline, evaluating a degree of absorption at a second wavelength spectrum of light transmitted through the combustible fuel, correlating the degree of absorption at the first wavelength spectrum and the degree of absorption at the second wavelength spectrum with a proportion of ethanol in the combustible fuel and a proportion of water in the combustible fuel; and outputting data corresponding to the proportions of ethanol and water in the combustible fuel to a controller of a combustion engine fed with the combustible fuel. The first and second wavelength spectrums consists of wavelengths of between about 800 nanometers (nm) and about 1200 nm.

In another example, a vehicle comprises a fuel tank that stores a combustible fuel including gasoline, an internal combustion engine that propels the vehicle, the internal combustion engine including a controller, a fuel line that carries the combustible fuel from the fuel tank to the internal combustion engine, a device positioned in-line with the fuel line. The device comprises a first optical channel that evaluates a degree of absorption at a first wavelength spectrum of light transmitted through the combustible fuel within the fuel line, and a second optical channel that evaluates a degree of absorption at a second wavelength spectrum of light transmitted through the combustible fuel within the fuel line. The first and second wavelength spectrums each consists of wavelengths of between about 800 nanometers (nm) and about 1200 nm. The device further comprises a controller configured to receive inputs from the first and second optical channels representing the degree of absorption at the first wavelength spectrum and the degree of absorption at the second wavelength spectrum, correlate the degree of absorption at the first wavelength spectrum and the degree of absorption at the second wavelength spectrum with a proportion of ethanol in the combustible fuel and a proportion of water in the combustible fuel, and output data corresponding to the proportions of ethanol and water in the combustible fuel to a controller of a combustion engine fed with the combustible fuel.

The details of one or more aspects of this disclosure are set forth in the accompanying drawings and the description below. Other features, objects, and advantages this disclosure will be apparent from the description and drawings, and from the claims.

### BRIEF DESCRIPTION OF DRAWINGS

FIG. 1 is a conceptual diagram of a device configured to determine proportions of ethanol and water in a combustible fuel including gasoline.

FIG. 2 is a conceptual diagram of a vehicle including an IC engine and the device of FIG. 1.

FIG. 3 is a graph illustrating light absorption of different gasoline-ethanol blends and ethanol-water blends over wavelength spectrum with a range of 800 nanometers (nm) to 1150 nm.

FIG. 4 is a graph illustrating light absorption of different gasoline-ethanol blends and ethanol-water blends over wavelength spectrum with a range of 1120 nm to 1180 nm.

FIG. 5 is a graph illustrating light absorption of different gasoline-ethanol blends over wavelength spectrum with a range of 200 nm to 450 nm.

FIGS. 6-8 are graphs illustrating light absorption of different commercially available gasolines over the wavelength spectrums of the graphs of FIGS. 3-5.

FIG. 9 is a flowchart illustrating techniques for determining proportions of ethanol and water in a combustible fuel including gasoline.

### DETAILED DESCRIPTION

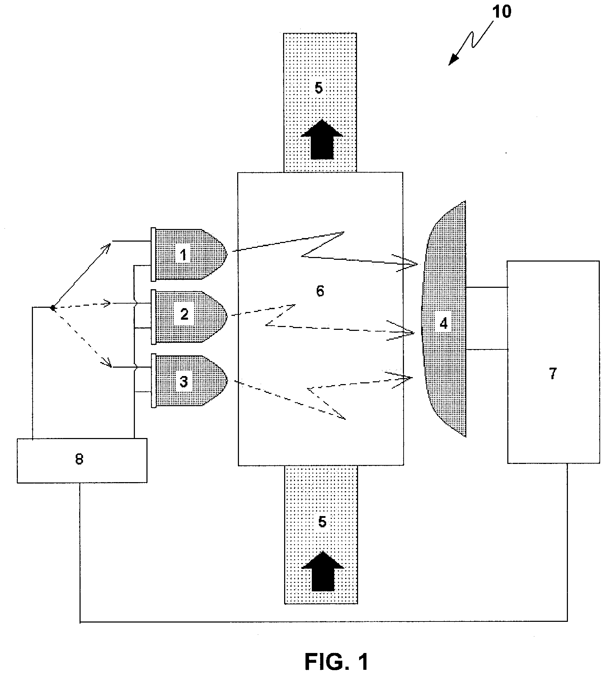

FIG. 1 is a conceptual diagram of device 10, which is configured to determine proportions of ethanol and water in a combustible fuel including gasoline. Device 10 includes fuel line 5 with detection chamber 6. Detection chamber is translucent and facilitates optical transmissions from light emitting diodes (LEDs) 1, 2, 3 to detector 4. LEDs 1, 2, 3 include reference LED 1, ethanol detection LED 2 and water detection LED 3, which are located on one side of detection chamber 6. Switch 8 selectively activates LEDs 1, 2, 3.

Detector 4 is located opposite LEDs 1, 2, 3, on an opposing side of detection chamber 6 and detects light emitted by LEDs 1, 2, 3 after the light passes through the combustible fuel in chamber 6. Detector 4 measures light intensity over a wavelength spectrum that includes the wavelength spectrums of light transmitted by LEDs 1, 2, 3. For example, detector 4 may measures light intensity over a wavelength spectrum within the NIR (near infra-red) range, such as a wavelength spectrum range from about 800 nm to about 1200 nm, a wavelength spectrum from about 800 nm to about 1100 nm, a wavelength spectrum from about 1100 nm to about 1200 nm or another wavelength spectrum. Notably, detectors and LEDs in the NIR range are generally less expensive than detectors and LEDs in the far infra-red range.

The distance between detector 4 and LEDs 1, 2, 3 affects the light intensity measured by detector 4. A greater distance means than more light is absorbed by the combustible fuel within chamber 6. The distance between detector 4 and LEDs 1, 2, 3 may be selected to provide sufficient sensitivity in order to determine proportions of ethanol and water in the fuel. In one example, such a distance may be between about 30 millimeters (mm) and about 80 mm. Other distances may also be used. The distance that provides the most desirable sensitivity may depend on the wavelength spectrums of LEDs 1, 2, 3 as well as the intensity of light emitted by LEDs 1, 2, 3.

When switch 8 turns on one of LEDs 1, 2, 3, light from the activated LED passes through detection chamber 6 and through liquid fuel located within chamber 6 before reaching detector 4. Because gasoline-ethanol blends with different proportions of ethanol and water absorb light differently, light intensities measured by detector 4 for each of LEDs 1, 2, 3 facilitate determining proportions of ethanol and water in a combustible fuel including gasoline located within chamber 6.

Controller 7 controls switch 8 and receives inputs from detector 4 corresponding to the measured light intensities from detector 4 to determine proportions of ethanol and water in the liquid fuel. Each of LEDs 1, 2, 3 provides a different wavelength spectrum. LEDs 1, 2, 3 combine with detector 4 to provide three distinct optical channels that evaluates a degree of absorption of the combustible fuel. For example, LEDs 2 and 3 may combine with detector 4 to provide first and second optical channels, whereas LED 1 may combine with detector 4 to provide a third optical channel. Each optical channel facilitates evaluation of a degree of absorption at a particular wavelength spectrum of light transmitted through the combustible fuel within fuel line 5.

In other examples, a device may provide optical channels using different techniques. For example, one or more of LEDs 1, 2, 3 may be replaced with laser light sources. Laser light sources can be configured to emit light in a more narrow wavelength spectrum than LEDs 1, 2, 3, which may provide for more precise determinations of the proportions of ethanol and water in the combustible fuel. As another example, a broad wavelength spectrum light source may be used in combination with optical filters or a prism to isolate particular wavelength spectrums to provide different optical channels. As yet another example, a device may include multiple detectors, each detector detecting a particular wavelength spectrum to provide different optical channels. In this manner, device 10 is merely exemplary, and other techniques for providing distinct optical channels are also within the spirit of this disclosure.

Controller 7 receives inputs from the optical channels representing the degree of absorption at wavelength spectrums provided by LEDs 1, 2, 3. Controller 7 then correlates the degree of absorption at wavelength spectrums provided by LEDs 1, 2, 3 with a proportion of ethanol in the combustible fuel and a proportion of water in the combustible fuel. Finally, controller 7 outputs data corresponding to the proportions of ethanol and water in the combustible fuel to a controller, such as a controller of a combustion engine fed with the combustible fuel.

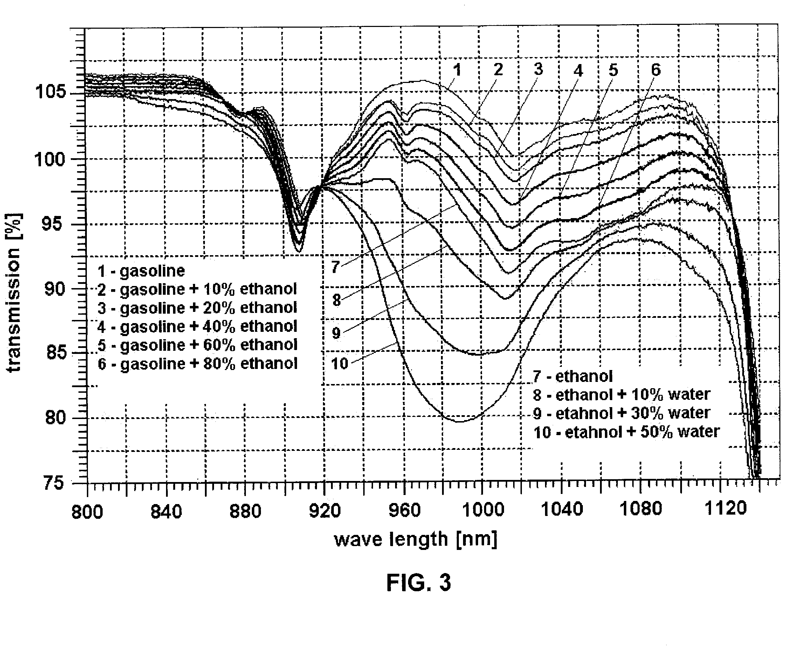

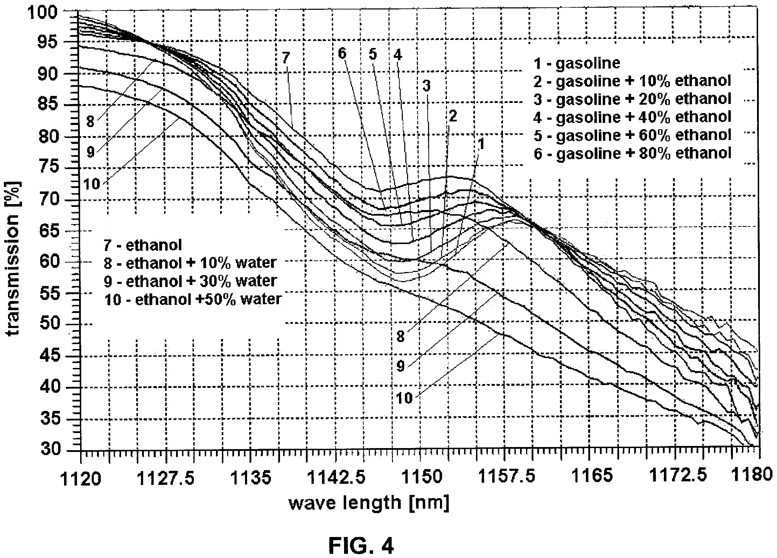

The wavelength spectrums of LEDs 1, 2, 3 are selected to facilitate calculation of the proportions of ethanol and water in a combustible fuel including gasoline located within chamber 6. Water detection LED 3 emits light at a wavelength spectrum selected to facilitate determination of the proportion of water in the combustible fuel. For example, as shown in FIG. 3, at wavelengths between about 920 nm to about 1020, and more particularly, between about 950 nm to about 1000 nm, the degree of absorption of the combustible fuel is predominately affected by the proportion of water and relatively less affected by the proportion of ethanol in the combustible fuel. As another example, as shown in FIG. 4, at wavelengths between about 1115 nm to about 1135 nm, and also at wavelengths between about 1155 nm to about 1170 nm, the degree of absorption of the combustible fuel is predominately affected by the proportion of water and relatively less affected by the proportion of ethanol. For this reason, water detection LED 3 may emit light at a wavelength spectrum centered within the range of about 920 nm to about 1020, and more particularly, between about 950 nm to about 1000 nm, within the range of about 1115 nm to about 1135 nm, or within the range of about about 1155 nm to about 1170 nm. As more specific examples, a wavelength spectrum centered around about 960 nm, about 1126 nm or about 1160 nm may be best suited to determine the proportion of water in the combustible fuel without needing to also determine the proportion of ethanol in the combustible fuel as the degree of absorption at these wavelengths is predominately affected by the proportion of water in the combustible fuel and relatively less affected by the proportion of ethanol in the combustible fuel.

Ethanol detection LED 2 emits light at a wavelength spectrum selected to facilitate determination of the proportion of ethanol in the combustible fuel. For example, as shown in FIG. 3, at wavelengths between about 1020 nm to about 1120, and more particularly, between about 1040 nm to about 1080 nm, the degree of absorption of the combustible fuel is predominately affected by the proportion of ethanol and relatively less affected by the proportion of water in the combustible fuel. For this reason, ethanol detection LED 2 may emit light at a wavelength spectrum centered within the range of about 1020 nm to about 1120, and more particularly, between about 1040 nm to about 1080 nm. As more specific example, a wavelength spectrum centered around about 1060 nm may be best suited to determine the proportion of ethanol in the combustible fuel without needing to also determine the proportion of water in the combustible fuel as the degree of absorption at 1060 nm is predominately affected by the proportion of ethanol in the combustible fuel and relatively less affected by the proportion of water in the combustible fuel.

As another example, as shown in FIG. 4, at wavelengths between about 1135 nm to about 1160 nm, the degree of absorption of the combustible fuel is significantly affected by both the proportion of ethanol and the proportion of water in the combustible fuel. Ethanol detection LED 2 may emit light at a wavelength spectrum centered within the range of about 1135 nm to about 1160 nm, or more specifically, centered within the range of about 1147 nm. In this example, controller 7 would need to combine a detected intensity of light transmitted by ethanol detection LED 2 with a detected intensity of light transmitted by water detection LED 3 to precisely determine the proportion of ethanol in the combustible fuel.

Likewise, at wavelengths between about 920 nm to about 1020, discussed above with respect to water detection LED 3, the degree of absorption of the combustible fuel is significantly affected by both the proportion of ethanol and the proportion of water in the combustible fuel. For this reason, the proportions of water within the combustible fuel may be more precisely determined if the light intensity detected from a water detection LED 3 that emits light within a wavelength spectrum centered within the range of about 920 nm to about 1020, is combined with the light intensity detected for ethanol detection LED 2.

In the event that the degree of absorption of the combustible fuel is significantly affected by both the proportion of ethanol and the proportion of water in the combustible fuel for the wavelength spectrums provided by either one or both of ethanol detection LED 2 and water detection LED 3, controller 7 may use the detected intensities of light transmitted by ethanol detection LED 2 and water detection LED 3 in combination to determine either the proportion of ethanol and/or the proportion of water in the combustible fuel. In some examples, controller 7 may first use the detected intensity of light transmitted by water detection LED 3 to separately determine the proportion of water in the combustible fuel before determining the proportion of ethanol in the combustible fuel. In other examples, controller 7 may simultaneously determine the proportion of ethanol in the combustible fuel and the proportion of water in the combustible fuel using a look-up table or equations representing the degree of absorption of the combustible fuel according to proportions of ethanol and water in the combustible fuel.

In summary, the two data points provided by ethanol detection LED 2 and water detection LED 3 may be used to simultaneously determine two unknowns, e.g., the proportion of ethanol in the combustible fuel and the proportion of water in the combustible fuel, even if the degree of absorption of the combustible fuel at the wavelength spectrum provided by ethanol detection LED 2 is dependent on both the proportion of ethanol in the combustible fuel and the proportion of water in the combustible fuel and if the degree of absorption of the combustible fuel at the wavelength spectrum provided by water detection LED 3 is also dependent on both the proportion of ethanol in the combustible fuel and the proportion of water in the combustible fuel. For this reason, it is not necessary that the degree of absorption at the wavelength spectrum provided by ethanol detection LED 2 be solely or even predominately affected by the proportion of ethanol in the combustible fuel and relatively less affected by the proportion of water in the combustible fuel. Nor is it necessary that the degree of absorption at the wavelength spectrum provided by water detection LED 3 be solely or even predominately affected by the proportion of water in the combustible fuel and relatively less affected by the proportion of ethanol in the combustible fuel.

Reference LED 1 emits light at a wavelength spectrum selected such that proportions of water and ethanol do not significantly affect the degree of absorption of a combustible fuel including gasoline located within chamber 6. For example, as shown in FIG. 3, wavelengths between about 800 nm and 920 nm, and more particularly, between about 800 nm and 850 nm, may provide approximately the same the degree of absorption for a combustible fuel including gasoline having any proportion of water and ethanol. For this reason, reference LED 1 may emit light at a wavelength spectrum centered within the range of about 800 nm and 920 nm, or within the range of about 800 nm and 850 nm.

Reference LED 1 may be suitable to calibrate detector 4 to determine the degree of absorption for light emitted by ethanol detection LED 2 and water detection LED 3. For example, variations in the temperature of the combustible fuel within chamber 6 can affect the degree of absorption for light emitted by ethanol detection LED 2 and water detection LED 3. Temperature variations can affect the measured degree of absorption up to five percent of the detected light transmission. To improve accuracy of device 10, the changes in degree of absorption due to temperature variation can be compensated by used of a third optical channel as provided by reference LED 1 and detector 4. As another example, the transparency of chamber 6 can be reduced over time, e.g., due to the flow of the combustible fuel, which also affects an intensity of light measured by detector 4. By emitting light within a wavelength spectrum having the same degree of absorption for any combustible fuel, reference LED 1 allows controller 7 to calibrate detector 4 for variations in the degree of absorption for combustible fuels at different temperatures temperature as well as variations in device that can occur over time, including but not limited to, changes in the translucence of chamber 6. In this manner, reference LED 1, allows controller 7 to more precisely determine the proportions of water and ethanol of a combustible fuel within chamber 6.

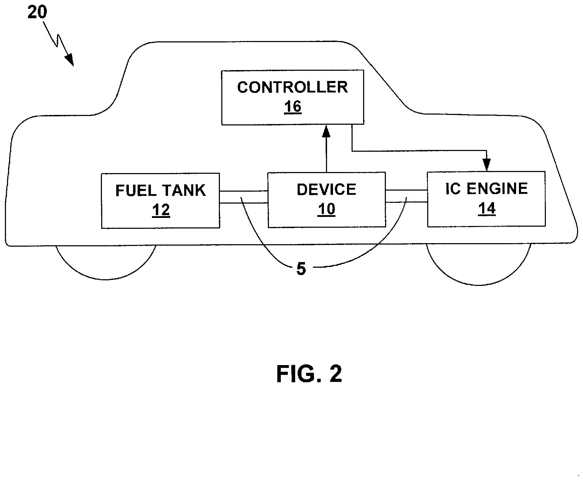

Controller 7 can be configured to output data corresponding to the proportions of ethanol and water in the combustible fuel to a controller of an IC engine. For example, as shown in FIG. 2, vehicle 20 includes IC engine 14 with IC engine controller 16. Vehicle 20 further includes fuel tank 12, and device 10 is positioned in-line with fuel line 5 between fuel tank 12 and IC engine 14. Controller 7 of device 10 outputs data corresponding to the proportions of ethanol and water in the combustible fuel to IC engine controller 16. IC engine controller 16 may adjust thus the ignition condition, e.g., air/fuel ratio and/or ignition timing, of IC engine 14 based on the data corresponding to the proportions of ethanol and water in the combustible fuel traversing fuel line 5, which can lead to reduce emissions improvements, increase efficiency, and/or increase reliability of IC engine 14.

In other examples, device 10 can be used to determine proportions of ethanol and water in any combustible fuel, such as a combustible fuel within a storage tank, pipeline or other location.

FIGS. 3-5 are graphs illustrating light absorption of different gasoline-ethanol blends and ethanol-water blends over different wavelength spectrums. Specifically, FIG. 3 is a graph illustrating light absorption of different gasoline-ethanol blends and ethanol-water blends over a wavelength spectrum with a range of 800 nm to 1150 nm, and FIG. 4 is a graph illustrating light absorption of different gasoline-ethanol blends and ethanol-water blends over wavelength spectrum with a range of 1120 nm to 1180 nm.

The plot lines illustrated in FIGS. 3 and 4 represent the transmission percent of light passed through the different gasoline-ethanol blends and ethanol-water blends at each wavelength in the spectrum. Transmission percent is inversely proportional to degree of absorption. The plot lines for ethanol-water blends are indicative the effect of water on the degree of absorption of light in gasoline-ethanol-water blends. For this reason, the plot lines for ethanol-water blends are suitable to select the wavelength spectrum of channels used to detect water in a combustible fuel including gasoline. It should be noted that water does not mix with gasoline, but only mixes with ethanol. Therefore, a combustible fuel could not consist of only water and gasoline as the gasoline would separate out.

As discussed above, FIG. 3 illustrates that at wavelengths between about 920 nm to about 1020, and more particularly, between about 950 nm to about 1000 nm, and even more specifically at about 960 nm, the degree of absorption of a combustible fuel is predominately affected by the proportion of water and relatively less affected by the proportion of ethanol in the combustible fuel. For example, spacing between lines 7-10, which represent varying ethanol-water blends is greater over these wavelengths than the spacing between lines 1-6, which represent various gasoline-ethanol blends.

FIG. 3 further illustrates that at wavelengths between about 1020 nm to about 1120, and more particularly, between about 1040 nm to about 1080 nm, and more specifically at about 1060 nm, the degree of absorption of the combustible fuel is predominately affected by the proportion of ethanol and relatively less affected by the proportion of water in the combustible fuel.

As further illustrated by FIG. 3, wavelengths between about 800 nm and 920 nm, and more particularly, between about 800 nm and 850 nm, provide approximately the same the degree of absorption for a combustible fuel including gasoline having any proportion of water and ethanol.

As also discussed above, FIG. 4 illustrates that at wavelengths between about 1115 5 nm to about 1135 nm, more particularly, about 1126 nm, and also at wavelengths between about 1155 nm to about 1170 nm, more particularly, about 1160 nm, the degree of absorption of the combustible fuel is predominately affected by the proportion of water and relatively less affected by the proportion of ethanol. FIG. 4 also illustrates that at wavelengths between about 1135 nm to about 1160 nm, more particularly, about 1147 nm, the degree of absorption of the combustible fuel is significantly affected by both the proportion of ethanol, but is also affected at least somewhat by the proportion of water in the combustible fuel.

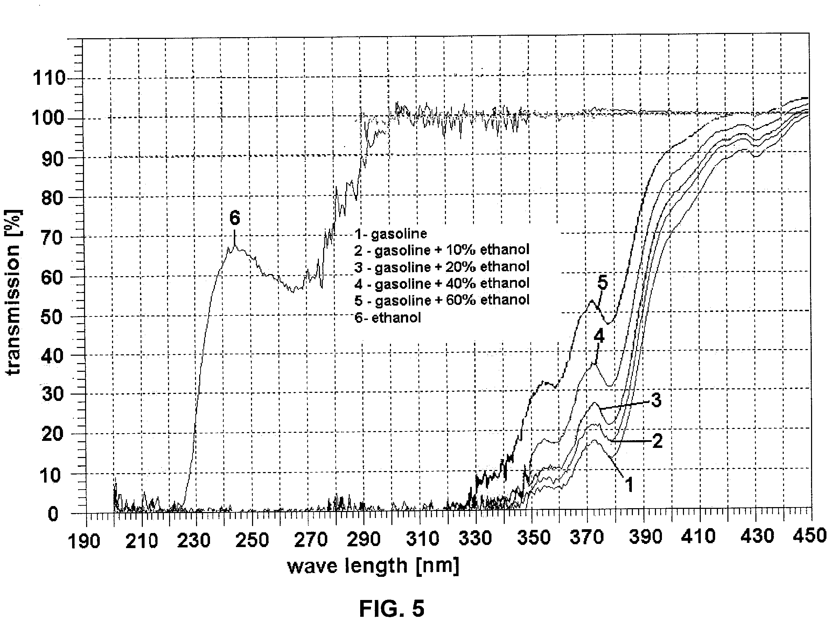

The plot lines illustrated in FIG. 5 represent the transmission percent of light passed through the different gasoline-ethanol blends over wavelength spectrum with a range of 200 nm to 450 nm (within the ultraviolet region). As previously mentioned, transmission percent is inversely proportional to degree of absorption. FIG. 5 illustrates that the degree of absorption of a gasoline-ethanol blend is highly-dependent on the proportion of ethanol at wavelengths between about 350 nm and about 420 nm.

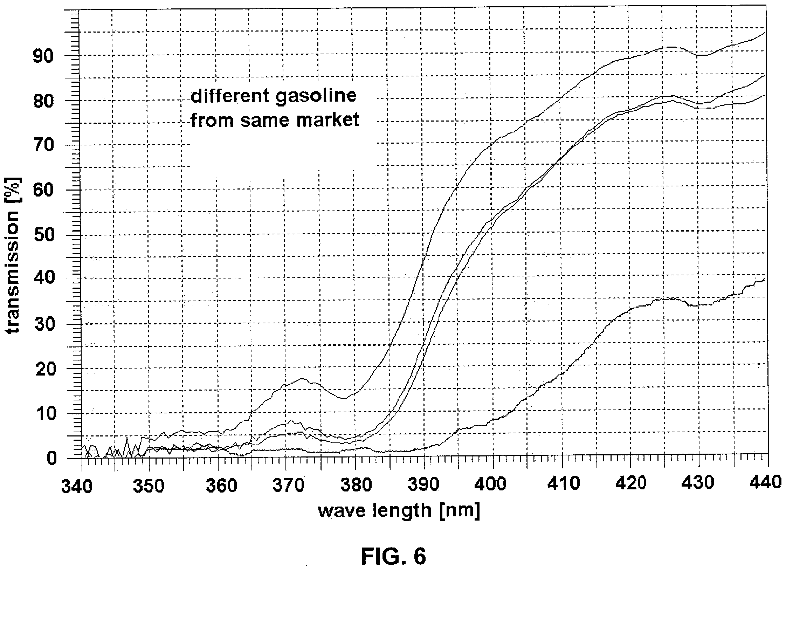

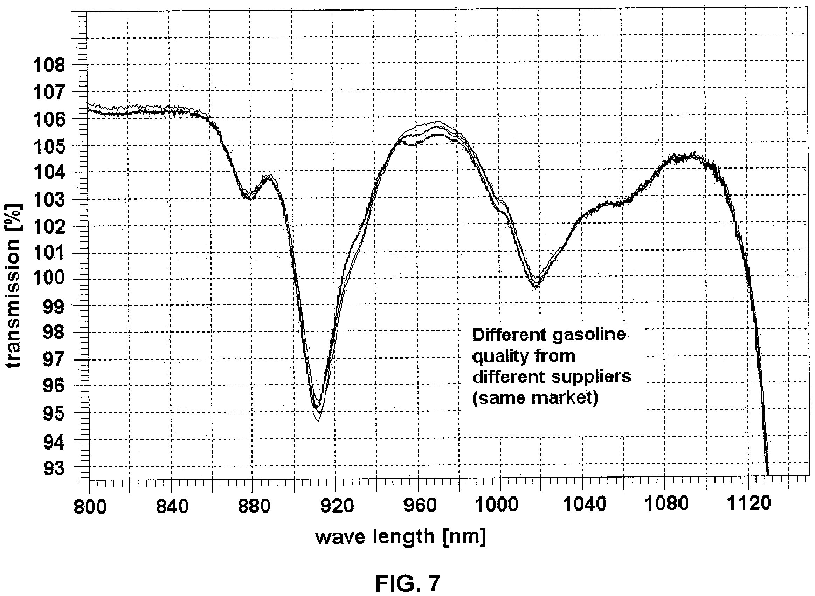

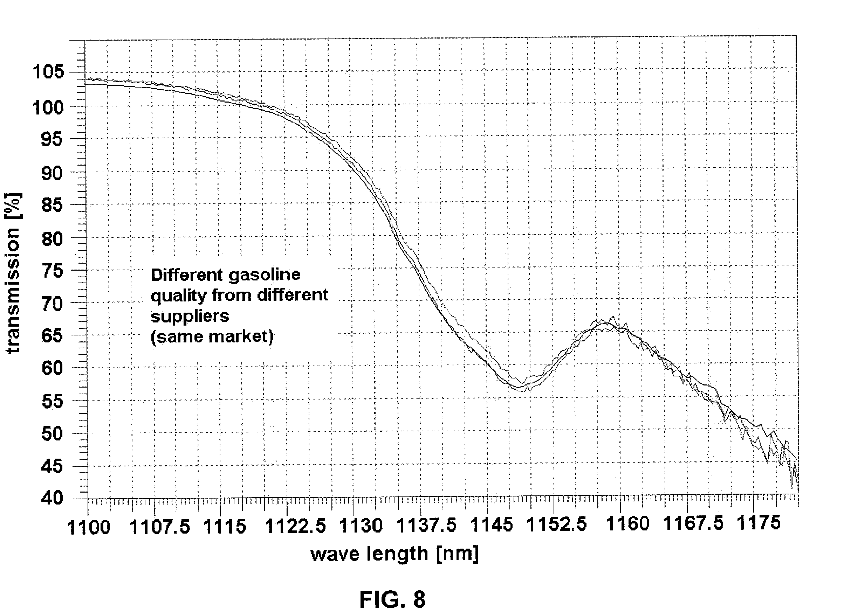

However, the graph of FIG. 6 illustrates that different commercial available gasolines provide greatly different degree of absorption at wavelengths within the ultraviolet region between 350 nm and about 440 nm. For this reason, wavelengths between about 350 nm and about 420 nm are not suitable for determining the proportion of ethanol in a gasoline-ethanol blend. In contrast, FIGS. 7 and 8 illustrate that the degree of absorption of different commercially available gasolines over the wavelength spectrum from about 800 nm to about 1100 nm (corresponding to FIG. 3) and the wavelength spectrum from about 1100 nm to about 1200 nm (corresponding to FIG. 4) are substantially similar for different commercially available gasolines. For this reason, the degree of absorption of wavelength spectrum consisting of wavelengths of between about 800 nm and about 1200 nm may be suitable for determining the proportions of ethanol and water in gasoline-ethanol blends.

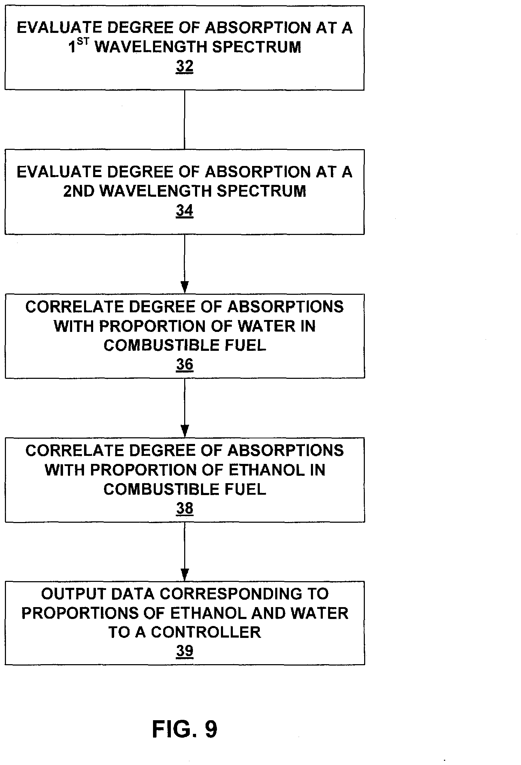

FIG. 9 is a flowchart illustrating techniques for determining proportions of ethanol and water in a combustible fuel including gasoline. As an example, the techniques shown in FIG. 9 may be performed by controller 7 of device 10 (FIG. 1).

First, controller 7 evaluates a degree of absorption at a first wavelength spectrum of light transmitted through a combustible fuel including gasoline (32). The first wavelength spectrum consists of wavelengths of between about 800 nm and about 1200 nm. Then controller 7 evaluates a degree of absorption at a second wavelength spectrum of light transmitted through the combustible fuel (34). The first wavelength spectrum consists of wavelengths of between about 800 nm and about 1200 nm.

Controller 7 correlates the degree of absorption at the first wavelength spectrum and the degree of absorption at the second wavelength spectrum with a proportion of ethanol in the combustible fuel and a proportion of water in the combustible fuel (36, 38). Controller 7 then outputs data corresponding to the proportions of ethanol and water in the combustible fuel to a controller, such as IC engine controller 14 (39).

The techniques described in this disclosure, such as techniques relating to controller 7 and IC controller 14, may be implemented, at least in part, in hardware, software, firmware or any combination thereof. For example, various examples of the techniques may be implemented within one or more microprocessors, digital signal processors (DSPs), application specific integrated circuits (ASICs), field programmable gate arrays (FPGAs), or any other equivalent integrated or discrete logic circuitry, as well as any combinations of such components, embodied in programmers, such as physician or patient programmers, stimulators, or other devices. The term "controller" may generally refer to any of the foregoing processors or logic circuitry, alone or in combination with other logic circuitry, or any other equivalent circuitry.

When implemented in software, the functionality ascribed to the systems and devices described in this disclosure may be embodied as instructions on a computer-readable storage medium such as random access memory (RAM), read-only memory (ROM), non-volatile random access memory (NVRAM), electrically erasable programmable read-only memory (EEPROM), FLASH memory, magnetic media, optical media, or the like. The instructions may be executed to cause one or more processors to support one or more examples of the functionality described in this disclosure.

Various aspects of the disclosure have been described. These and other aspects are within the scope of the following claims.

## Claims

1. A device comprising:
a fuel line that carries a combustible fuel including gasoline;
a first optical channel that evaluates a degree of absorption at a first wavelength spectrum of light transmitted through the combustible fuel within the fuel line, wherein the first wavelength spectrum consists of wavelengths of between about 800 nanometers (nm) and about 1200 nm;
a second optical channel that evaluates a degree of absorption at a second wavelength spectrum of light transmitted through the combustible fuel within the fuel line, wherein the second wavelength spectrum consists of wavelengths of between about 800 nm and about 1200 nm; and
a controller configured to:
receive inputs from the first and second optical channels representing the degree of absorption at the first wavelength spectrum and the degree of absorption at the second wavelength spectrum;
correlate the degree of absorption at the first wavelength spectrum and the degree of absorption at the second wavelength spectrum with a proportion of ethanol in the combustible fuel and a proportion of water in the combustible fuel; and
output data corresponding to the proportions of ethanol and water in the combustible fuel to a controller of a combustion engine fed with the combustible fuel.

2. The device of claim 1, wherein the device includes:
a first light source that emits light at the first wavelength spectrum into the fuel line;
a second light source that emits light at the second wavelength spectrum into the fuel line; and
a detector that detects light emitted by the first light source and the second light source after the light passes through the fuel line,
wherein first optical channel includes the first light source and the detector, and
wherein second optical channel includes the second light source and the detector.

3. The device of any of claims 1 or 2, further comprising a third optical channel that evaluates an intensity of light transmitted through the combustible fuel within the fuel line at a third wavelength spectrum, wherein the intensity of light transmitted through the combustible fuel including gasoline at the third wavelength spectrum is relatively unaffected by proportions of water and ethanol in the combustible fuel,
wherein the controller is further configured to:
receive input from the third optical channel representing the intensity of light transmitted through the combustible fuel within the fuel line at the third wavelength spectrum; and
determine the degree of absorption at the first wavelength spectrum and the degree of absorption at the second wavelength spectrum based on the inputs from the first, second and third optical channels.

4. The device of any of claims 1 - 3,
wherein the degree of absorption at the first wavelength spectrum is predominately affected by the proportion of ethanol in the combustible fuel and relatively less affected by the proportion of water in the combustible fuel, and
wherein the degree of absorption at the second wavelength spectrum is predominately affected by the proportion of water in the combustible fuel and relatively less affected by the proportion of ethanol in the combustible fuel.

5. The device of any of claims 1 - 4, wherein the first wavelength spectrum is centered within a range selected from a group consisting of:
about 1020 nm to about 1120 nm;
about 1040 nm to about 1080 nm; and
about 1135 nm to about 1160 nm.

6. The device of any of claims 1 - 5, wherein the second wavelength spectrum is centered within a range selected from a group consisting of:
about 920 nm to about 1020 nm;
about 950 nm to about 1000 nm;
about 1115 nm to about 1135 nm; and
about 1155 nm to about 1170 nm.

7. A method comprising:
evaluating a degree of absorption at a first wavelength spectrum of light transmitted through a combustible fuel including gasoline, wherein the first wavelength spectrum consists of wavelengths of between about 800 nanometers (nm) and about 1200 nm;
evaluating a degree of absorption at a second wavelength spectrum of light transmitted through the combustible fuel, wherein the first wavelength spectrum consists of wavelengths of between about 800 nm and about 1200 nm;
correlating the degree of absorption at the first wavelength spectrum and the degree of absorption at the second wavelength spectrum with a proportion of ethanol in the combustible fuel and a proportion of water in the combustible fuel; and
outputting data corresponding to the proportions of ethanol and water in the combustible fuel to a controller.

8. The method of claim 7,
wherein the degree of absorption at the first wavelength spectrum is predominately affected by the proportion of ethanol in the combustible fuel and relatively less affected by the proportion of water in the combustible fuel, and
wherein the degree of absorption at the second wavelength spectrum is predominately affected by the proportion of water in the combustible fuel and relatively less affected by the proportion of ethanol in the combustible fuel.

9. The method of any of claims 7 or 8, wherein evaluating the degrees of absorption at the first and second wavelength spectrums include, evaluating an intensity of light transmitted through the combustible fuel including gasoline at a third wavelength spectrum, wherein the intensity of light transmitted through the combustible fuel including gasoline at the third wavelength spectrum is relatively unaffected by proportions of water and ethanol in the combustible fuel.

10. The method of any of claims 7 - 9,
wherein the first wavelength spectrum is centered within a range selected from a first group consisting of:
about 1020 nm to about 1120 nm;
about 1040 nm to about 1080 nm; and
about 1135 nm to about 1160 nm, and
wherein the second wavelength spectrum is centered within a range selected from a group consisting of:
about 920 nm to about 1020 nm;
about 950 nm to about 1000 nm;
about 1115 nm to about 1135 nm; and
about 1155 nm to about 1170 nm.
